# EUROPEAN PATENT APPLICATION

(11) **EP 4 397 233 A1**
(43) Date of publication of application: **10.07.2024**
(21) Application number: 23858416.3
(22) Date of filing: 18.04.2023
(51) Int. Cl.: A61B 5/0225, G06N 3/04, G06V 30/14

(54) **PROCESS CONTROL SYSTEM AND IMPLEMENTATION METHOD FOR STANDARDIZED BLOOD PRESSURE MEASUREMENT**

(30) Priority: 21.11.2022 CN 202211451732
(71) Applicant: Jiangxi Changgang Medical Technology Limited Company, Nanchang, Jiangxi 330038 (CN)
(72) Inventor: XIA, Linglin, Nanchang, Jiangxi 330038 (CN); ZHANG, Yuyuan, Nanchang, Jiangxi 330038 (CN); YUAN, Aihua, Nanchang, Jiangxi 330038 (CN); GENG, Renliang, Nanchang, Jiangxi 330038 (CN); SU, Hai, Nanchang, Jiangxi 330038 (CN)
(74) Representative: Sach, Greg Robert
(86) International application number: PCT/CN2023/088819
(87) International publication number: WO 2024/108881

(57) **Abstract**

A process control system for standardized blood pressure measurement and implementation method, includes a blood pressure measurement process control device and a built-in control module, the blood pressure measurement process control device includes a computer host, a display screen, a control switch and an electronic sphygmomanometer, the electronic sphygmomanometer is mounted inside the blood pressure measurement process control device through a fixed structure. The application realizes a group of standardized blood pressure measurements for patients by controlling the electronic sphygmomanometer through the built-in each control module, automatically completes binding and outputting patient's identity information and blood pressure measurement results, and generates a blood pressure measurement report with complete information, so as to reduce the work intensity of medical staff, meanwhile reduce the measurement procedural error caused by human factors, and improve the reliability of measurement results and records.

## Description

### TECHNICAL FIELD

The present application relates to the technical field of process control of blood pressure measurement, in particular to a process control system for standardized blood pressure measurement and implementation method.

### BACKGROUND

Modern medicine mostly uses electronic sphygmomanometers to complete the blood pressure measurement of the human body. However, the measurement process of traditional manual control electronic sphygmomanometers is especially arbitrary, and different medical staff will obtain significantly different blood pressure measurement results for the same subject, indicating obvious measurement procedural errors; at the same time, there are also shortcomings in the data collection and management of measurement results. For example, the electronic sphygmomanometer itself only has the function of blood pressure measurement, and does not have the function of entering and binding the subject's identity information. When using it, medical staff usually need to manually record the subject's identity information, which is prone to errors and increases the workload of medical staff. Even an electronic sphygmomanometer with data storage function can only store measurement data in chronological order, the entry of the subject's identity information still needs to be completed manually, and when there are many subjects, it is also easy to cause confusion between the subject's identity information and their blood pressure measurement data, and the blood pressure data of the subject and that of other subjects will appear mutual confusion.

### SUMMARY

In view of the above problems, the present application proposes a process control system for standardized blood pressure measurement, which realizes a group of standardized blood pressure measurements (bilateral synchronization) for patients by controlling the electronic sphygmomanometer, and automatically completes binding and outputting patient's identity information and blood pressure measurement results, while reducing the work intensity of medical staff, reduce the measurement procedural error caused by human factors, and improve the reliability of measurement results and records.

In order to achieve the above purpose, the present application adopts the following technical solutions:

A process control system for standardized blood pressure measurement, wherein includes a blood pressure measurement process control device and a built-in control module, the blood pressure measurement process control device includes a computer host, a display screen, a control switch and an electronic sphygmomanometer, and the computer host is used to control the entire measurement process, and the display and output of the results; the display screen is used for human-computer interaction and display of the measurement results; the control switch is used to control the startup and shutdown of the electronic sphygmomanometer; the electronic sphygmomanometer is used to realize blood pressure measurement, and is mounted inside the blood pressure measurement process control device through a fixed structure;
The control module includes an information acquisition module, an image clarity detection module, a data processing module and a blood pressure measurement process control module, wherein the information acquisition module is used to take pictures of the user's identity document information and the measurement results on the display panel of the electronic sphygmomanometer each time, and send the collected image to the image clarity detection module; the image clarity detection module is used to carry out the clarity detection for the collected image, and send the qualified image to the data processing module; the data processing module includes an image preprocessing module, a text detection module, a text recognition module and a regex filtering module, the image preprocessing module is used to crop the document image according to specifications and correct the image taken by the electronic sphygmomanometer display panel, the text detection module and the text recognition module are used to detect and recognize the textual information in the document image and the blood pressure measurement result image, the regex filtering module extracts and fills the key information of the text by specifying the corresponding regex filtering standard, calculates the average and difference values of the blood pressure measurement readings, integrates the user's identity information with the blood pressure measurement results, and then displays it on the display screen.

Further, the control command signal of the control switch is regularly sent out by the built-in program of the signal acquisition module, and the virtual switch is driven by the I/O control card connected through the USB interface to replace the traditional manual start button.

Specifically, the implementation method of the process control system for standardized blood pressure measurement includes the following steps:
Step S1. System environment configuration: placing the blood pressure measurement process control device on a horizontal desktop, connecting the power supply and turning on the power switch of the combined device, and the control system entering a standby state;
Step S2. Identity information acquisition: the user places the identity card or medical insurance card at the designated photo location, and the information acquisition module controls the camera to take photos and samples the user's identity document information, and after the collected photos are qualified by the image clarity detection module, they are sent to the data processing module to generate a list of identity information;
Step S3. Data acquisition and processing: after the identity information is successfully collected, the blood pressure measurement control module controls the electronic sphygmomanometer to complete the user's blood pressure measurement, and the information acquisition module controls the camera to sample the data displayed on the display panel of the electronic sphygmomanometer each time; the data processing module identifies and extracts the sampled data, and further calculates the average and difference values of the blood pressure measurement results to obtain a list of blood pressure measurement results;
Step S4. Measurement report synthesis, printing, and uploading: the data processing module integrates the list of identity information generated in the step S2 with the list of blood pressure measurement results in the step S3, displays it on the display screen, and uploads it to the hospital HIS system or cloud server for storage.

Further, the specific process of the identity information acquisition in the step S2 is as follows:
Step S21. Document image acquisition: the user places the identity card or medical insurance card at the designated photo location, and the information acquisition module controls the camera to take photos, and then the image clarity detection module detects the clarity of the image. If the pixel value of the collected document image is less than the set threshold value of 1080x960, an error message will be returned to prompt the user to re-take and sample a photo; if the pixel value of the collected document image is greater than or equal to the set threshold value of 1080x960, then step S32 will be executed;
Step S22. Document image processing: the image preprocessing module uniformly crops the document images collected in step S31 to an image with an aspect ratio of 3:2 and performs grayscale processing;
Step S23. Document image recognition: using the text detection module and the text recognition module to detect and recognize the textual information in the processed document image, and then obtain the text recognition result of the image after regex filtering;
Step S24. Document information storage and binding: according to the text recognition result obtained in step S33, storing the user's name, gender, and age information to generate a list of identity information, and outputting a blood pressure measurement report after binding with the subsequent list of blood pressure measurement results.

The specific process of the data acquisition and processing is as follows:
Step S31. Display panel image acquisition: the information acquisition module controls the camera to collect photos of the display panel of the electronic sphygmomanometer at the designated location, and after the collected photos are qualified by the image clarity detection module, they are sent to the data processing module;
Step S32. Panel image correction: the image preprocessing module performs further corner detection and affine transformation on the collected image to realize image correction;
Step S33. Character segmentation, recognition and result calculation: the text detection module and the text recognition module complete the independent segmentation of the characters in the corrected image, and use the threading method to perform character recognition on the independently segmented image, and then use the regex filtering module to extract the key information from the recognition text, and calculates the average and difference values of the blood pressure measurement readings.

Further, the specific process of using the text detection module and the text recognition module to detect and recognize the textual information in the processed document image in the step S23 is as follows:
Text detection: using the DBnet network to realize text detection, outputting the text segmentation result of the image through the network based on the segmentation method, using the preset threshold T to convert the segmentation result image into a binary image, inserting the image binarization operation into the network for j oint optimization, through more than 4 sampling operations, the network adaptively predicts the threshold of each pixel point in the image, using the ICPR dataset to train the document information model, parsing the text into a list and storing it, then performing recognition after training;
Text recognition: the recognition algorithm is implemented by CRNN+CTC, by extracting features, the text sequence features are extracted based on convolution features, and selecting GRU with lower computational cost; CNN uses Mobilenet to scale to the size of 32x640x32 under the case of fixed aspect ratio of the image, which becomes 1x8x512 after passing through the CNN; setting T=8 for LSTM, and inputting the features into LSTM, in the actual recognition process, taking into account the text repetition, interruption, and different intervals of document information, using CTC to output the results, borrowing the forward-backward algorithm of HMM to predict the blanks in the text by human-specified blank symbols, finally enumerating all possible results, and outputting the recognition text information.

Further, the specific process of using the preset threshold T to convert the segmentation result image into a binary image and the threshold T is obtained by using the OTSU threshold segmentation algorithm is as follows:
① The size of the blood pressure measurement result image P is width w, height h, the segmentation threshold is T, and T is traversed from 0 to 255;
② Dividing the image into two parts according to the size of T, and the number of pixels smaller than the threshold T is N₀ and the number of pixels greater than the threshold T is N₁, wherein N₀ + *N₁* = *w* * *h;*
(3) The proportions of the two divided parts to the entire image P are *ω*₀ and *ω*₁, *ω*₀ + *ω*₁ = 1; the average gray level are *µ*₀ and *µ*₁ separately, wherein *µ*₀ + *µ*₁ = 1;
(4) The average gray value of the image P is *µ*, with a variance of *ε*, *ε* = *ω*₀(*µ*₀ - µ)² + *ω*₁(*µ*₁ - *µ*)², according to the OTSU algorithm, it can be derived:
   *ε* = *ω*₀*ω*₁(µ₀ - µ₁*)²* , wherein *ω*₀ = *N₀*/*(w* * *h), ω₁* = *N₁*/*(w* * *h), µ* = *µ*₀*ω*₀ + *µ*₁*ω*₁;
   By traversing the pixels of the image P, determining the size of the threshold T, that is, the size of the threshold T based on the maximum value of the variance ε.

The beneficial effects of the present application are:
1. The present application proposes a process control system for standardized blood pressure measurement, which realizes a group of standardized blood pressure measurements (bilateral synchronization) for patients by controlling the electronic sphygmomanometer, it can reduce the measurement procedural error caused by human factors and obtain more comprehensive information, improve the credibility of the measurement results, and help doctors make more reasonable judgments on the subsequent diagnosis and treatment of hypertensive patients.
2. The present application proposes an implementation method of a process control system for standardized blood pressure measurement, which can automatically complete binding and outputting patient's identity information and blood pressure measurement results, it can provide convenient and reliable standardized blood pressure measurement process control means and corresponding data management capability, reduce the workload of medical staff.

### BRIEF DESCRIPTION OF THE DRAWINGS

FIG. 1 is a structural block diagram of a standardized double-arm/ankle synchronous blood pressure measurement system of the present application;
FIG. 2 is a flow chart of a standardized double-arm/ankle synchronous blood pressure measurement method of the present application;
FIG. 3 is a flow chart of information interaction provided by embodiments of the present application;
FIG. 4 is a flow chart of image processing provided by embodiments of the present application.

### DETAILED DESCRIPTION OF THE EMBODIMENTS

In order to make those skilled in the art to better understand the technical solutions in this specification, the technical solutions in the embodiments of this specification will be clearly and completely described below in conjunction with the drawings in the embodiments of this specification. Obviously, the described embodiments are only some of the embodiments of the present application, but not all of them.

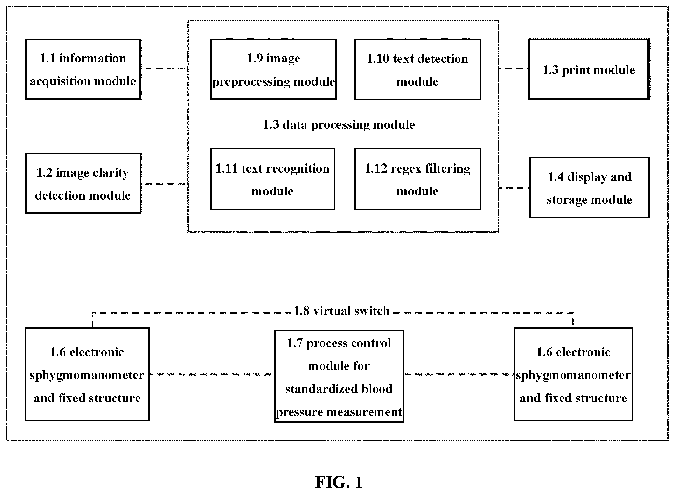

Referring to FIG. 1, a process control system for standardized blood pressure measurement, wherein includes a blood pressure measurement process control device and a built-in control module, the blood pressure measurement process control device includes a computer host, a display screen, a control switch and an electronic sphygmomanometer, and the computer host is used to control the entire measurement process, and the display and output of the results; the display screen is used for human-computer interaction and display of the measurement results; the control switch is used to control the startup and shutdown of the electronic sphygmomanometer; the electronic sphygmomanometer is used to realize blood pressure measurement, and is mounted inside the blood pressure measurement process control device through a fixed structure;

The control module includes an information acquisition module, an image clarity detection module, a data processing module and a blood pressure measurement process control module, wherein the information acquisition module is used to take pictures of the user's identity document information and the measurement results on the display panel of the electronic sphygmomanometer each time, and send the collected image to the image clarity detection module; the image clarity detection module is used to carry out the clarity detection for the collected image, and send the qualified image to the data processing module; the data processing module includes an image preprocessing module, a text detection module, a text recognition module and a regex filtering module, the image preprocessing module is used to crop the document image according to specifications and correct the image taken by the electronic sphygmomanometer display panel, the text detection module and the text recognition module are used to detect and recognize the textual information in the document image and the blood pressure measurement result image, the regex filtering module extracts and fills the key information of the text by specifying the corresponding regex filtering standard, calculates the average and difference values of the blood pressure measurement readings, integrates the user's identity information with the blood pressure measurement results, and then displays it on the display screen.

Specifically, the control command signal of the control switch is regularly sent out by the built-in program of the signal acquisition module, and the virtual switch is driven by the I/O control card connected through the USB interface to replace the traditional manual start button.

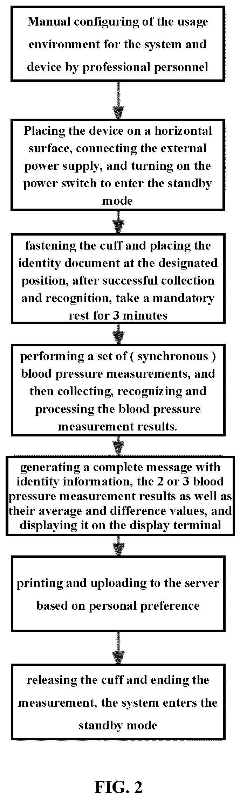

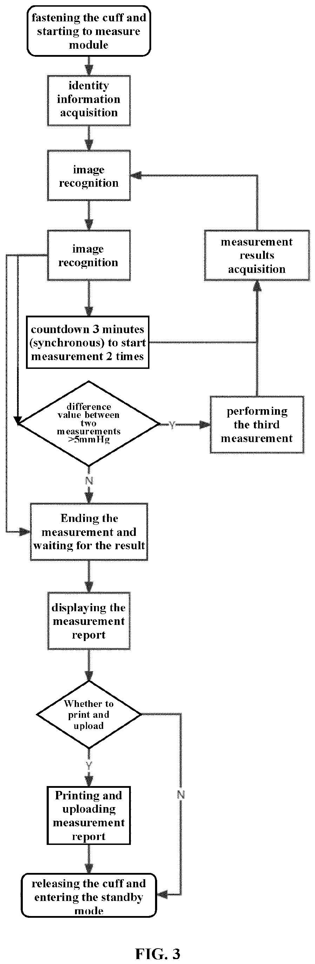

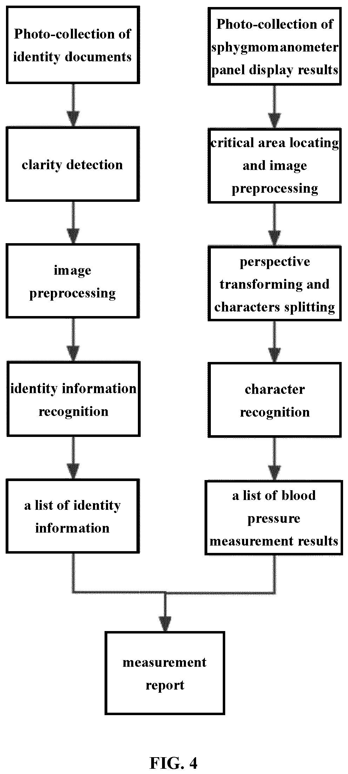

Further, referring to FIG.2- FIG.4, the implementation method of the process control system for standardized blood pressure measurement is also provided in this embodiment, including the following steps:
Step S1. System environment configuration: placing the blood pressure measurement process control device on a horizontal desktop, connecting the power supply and turning on the power switch of the combined device, and the control system entering a standby state;
Step S2. Identity information acquisition: the user places the identity card or medical insurance card at the designated photo location, and the information acquisition module controls the camera to take photos and samples the user's identity document information, and after the collected photos are qualified by the image clarity detection module, they are sent to the data processing module to generate a list of identity information;
Step S3. Data acquisition and processing: after the identity information is successfully collected, the blood pressure measurement control module controls the electronic sphygmomanometer to complete the user's blood pressure measurement, and the information acquisition module controls the camera to sample the data displayed on the display panel of the electronic sphygmomanometer each time; the data processing module identifies and extracts the sampled data, and further calculates the average and difference values of the blood pressure measurement results to obtain a list of blood pressure measurement results;
Step S4. Measurement report synthesis, printing, and uploading: the data processing module integrates the list of identity information generated in the step S2 with the list of blood pressure measurement results in the step S3, displays it on the display screen, and uploads it to the hospital HIS system or cloud server for storage.

Further, the specific process of the identity information acquisition in the step S2 is as follows:
Step S21. Document image acquisition: the user places the identity card or medical insurance card at the designated photo location, and the information acquisition module controls the camera to take photos, and then the image clarity detection module detects the clarity of the image. If the pixel value of the collected document image is less than the set threshold value of 1080x960, an error message will be returned to prompt the user to re-take and sample a photo; if the pixel value of the collected document image is greater than or equal to the set threshold value of 1080x960, then step S32 will be executed;
Step S22. Document image processing: the image preprocessing module uniformly crops the document images collected in step S31 to an image with an aspect ratio of 3:2 and performs grayscale processing;
Step S23. Document image recognition: using the text detection module and the text recognition module to detect and recognize the textual information in the processed document image, and then obtain the text recognition result of the image after regex filtering;
Step S24. Document information storage and binding: according to the text recognition result obtained in step S33, storing the user's name, gender, and age information to generate a list of identity information, and outputting a blood pressure measurement report after binding with the subsequent list of blood pressure measurement results.

The specific process of the data acquisition and processing is as follows:
Step S31. Display panel image acquisition: the information acquisition module controls the camera to collect photos of the display panel of the electronic sphygmomanometer at the designated location, and after the collected photos are qualified by the image clarity detection module, they are sent to the data processing module;
Step S32. Panel image correction: the image preprocessing module performs further corner detection and affine transformation on the collected image to realize image correction;
Step S33. Character segmentation, recognition and result calculation: the text detection module and the text recognition module complete the independent segmentation of the characters in the corrected image, and use the threading method to perform character recognition on the independently segmented image, and then use the regex filtering module to extract the key information from the recognition text, and calculates the average and difference values of the blood pressure measurement readings.

Further, the specific process of using the text detection module and the text recognition module described in the step S23 to detect and recognize the textual information in the processed document image is as follows:
Text detection: using the DBnet network to realize text detection, outputting the text segmentation result of the image through the network based on the segmentation method, using the preset threshold T to convert the segmentation result image into a binary image, inserting the image binarization operation into the network for oint optimization, through more than 4 sampling operations, the network adaptively predicts the threshold of each pixel point in the image, using the ICPR dataset to train the document information model, parsing the text into a list and storing it, then performing recognition after training;
Text recognition: the recognition algorithm is implemented by CRNN+CTC, by extracting features, the text sequence features are extracted based on convolution features, and selecting GRU with lower computational cost; CNN uses Mobilenet to scale to the size of 32x640x32 under the case of fixed aspect ratio of the image, which becomes 1x8x512 after passing through the CNN; setting T=8 for LSTM, and inputting the features into LSTM, in the actual recognition process, taking into account the text repetition, interruption, and different intervals of document information, using CTC to output the results, borrowing the forward-backward algorithm of HMM to predict the blanks in the text by human-specified blank symbols, finally enumerating all possible results, and outputting the recognition text information.

Further, the specific process of using the preset threshold T to convert the segmentation result image into a binary image and the threshold T is obtained by using the OTSU threshold segmentation algorithm is as follows:
① The size of the blood pressure measurement result image P is width w, height h, the segmentation threshold is T, and T is traversed from 0 to 255;
② Dividing the image into two parts according to the size of T, and the number of pixels smaller than the threshold T is *N*₀ and the number of pixels greater than the threshold T is *N*₁, wherein *N*₀ + *N₁* = *w* * *h;*
(3) The proportions of the two divided parts to the entire image P are *ω*₀ and *ω*₁, *ω*₀ + *ω*₁ = 1; the average gray level are *µ*₀ and *µ*₁ separately, wherein *µ*₀ + *µ*₁ = 1;
(4) The average gray value of the image P is *µ*, with a variance of *ε*, *ε* = *ω*₀(*µ*₀ - *µ*)² + *ω*₁(*µ*₁ - *µ*)², according to the OTSU algorithm, it can be derived:
   *ε* = *ω*₀*ω*₁(*µ*₀ - µ₁*)²* , wherein *ω*₀ = *N₀*/(*w* * *h*), *ω₁* = *N₁*/(*w* * *h*), *µ* = *µ*₀*ω*₀ + *µ*₁*ω*₁;
   By traversing the pixels of the image P, determining the size of the threshold T, that is, the size of the threshold T based on the maximum value of the variance *ε*.

The preferred implementation method of the present application has been specifically described above, but the application is not limited to the described embodiments, and those skilled in the art can also make various equivalent variations or substitutions without violating the spirit of the present application. These equivalent variations or substitutions are all included in the scope defined by the claims of the present application.

## Claims

1. A process control system for standardized blood pressure measurement, comprising a blood pressure measurement process control device and a built-in control module, wherein the blood pressure measurement process control device comprises a computer host, a display screen, a control switch and an electronic sphygmomanometer, and the computer host is configured to control the entire measurement process, and the display and output of the results; the display screen is configured for human-computer interaction and display of the measurement results; the control switch is configured to control the startup and shutdown of the electronic sphygmomanometer; the electronic sphygmomanometer is configured to realize blood pressure measurement, and is mounted inside the blood pressure measurement process control device through a fixed structure;
the control module comprises an information acquisition module, an image clarity detection module, a data processing module and a blood pressure measurement process control module, wherein the information acquisition module is configured to take pictures of the user's identity document information and the measurement results on the display panel of the electronic sphygmomanometer each time, and send the collected image to the image clarity detection module; the image clarity detection module is configured to carry out the clarity detection for the collected image, and send the qualified image to the data processing module; the data processing module comprises an image preprocessing module, a text detection module, a text recognition module and a regex filtering module, the image preprocessing module is configured to crop the document image according to specifications and correct the image taken by the electronic sphygmomanometer display panel, the text detection module and the text recognition module are configured to detect and recognize the textual information in the document image and the blood pressure measurement result image, the regex filtering module extracts and fills the key information of the text by specifying the corresponding regex filtering standard, calculates the average and difference values of the blood pressure measurement readings, integrates the user's identity information with the blood pressure measurement results, and then displays it on the display screen.

2. The system of claim 1, wherein the control command signal of the control switch is regularly sent out by the built-in program of the signal acquisition module, and the virtual switch is driven by the I/O control card connected through the USB interface to replace the traditional manual start button.

3. A implementation method of the process control system for standardized blood pressure measurement, comprising:
Step S1: system environment configuration: placing the blood pressure measurement process control device on a horizontal desktop, connecting the power supply and turning on the power switch of the combined device, and the control system entering a standby state;
Step S2: identity information acquisition: the user places the identity card or medical insurance card at the designated photo location, and the information acquisition module controls the camera to take photos and samples the user's identity document information, and after the collected photos are qualified by the image clarity detection module, they are sent to the data processing module to generate a list of identity information;
Step S3: data acquisition and processing: after the identity information is successfully collected, the blood pressure measurement control module controls the electronic sphygmomanometer to complete the user's blood pressure measurement, and the information acquisition module controls the camera to sample the data displayed on the display panel of the electronic sphygmomanometer each time; the data processing module identifies and extracts the sampled data, and further calculates the average and difference values of the blood pressure measurement results to obtain a list of blood pressure measurement results;
Step S4: measurement report synthesis, printing, and uploading: the data processing module integrates the list of identity information generated in the step S2 with the list of blood pressure measurement results in the step S3, displays it on the display screen, and uploads it to the hospital HIS system or cloud server for storage.

4. The method of claim 1, wherein the specific process of the identity information acquisition in the step S2 is as follows:
Step S21: document image acquisition: the user places the identity card or medical insurance card at the designated photo location, and the information acquisition module controls the camera to take photos, and then the image clarity detection module detects the clarity of the image, if the pixel value of the collected document image is less than the set threshold value of 1080x960, an error message will be returned to prompt the user to re-take and sample a photo; if the pixel value of the collected document image is greater than or equal to the set threshold value of 1080x960, then step S32 will be executed;
Step S22: document image processing: the image preprocessing module uniformly crops the document images collected in step S31 to an image with an aspect ratio of 3:2 and performs grayscale processing;
Step S23: document image recognition: using the text detection module and the text recognition module to detect and recognize the textual information in the processed document image, and then obtain the text recognition result of the image after regex filtering;
Step S24: document information storage and binding: according to the text recognition result obtained in step S33, storing the user's name, gender, and age information to generate a list of identity information, and outputting a blood pressure measurement report after binding with the subsequent list of blood pressure measurement results.

5. The method of claim 1, wherein the specific process of the data acquisition and processing in the step S3 is as follows:
Step S31: display panel image acquisition: the information acquisition module controls the camera to collect photos of the display panel of the electronic sphygmomanometer at the designated location, and after the collected photos are qualified by the image clarity detection module, they are sent to the data processing module;
Step S32: panel image correction: the image preprocessing module performs further corner detection and affine transformation on the collected image to realize image correction;
Step S33: character segmentation, recognition and result calculation: the text detection module and the text recognition module complete the independent segmentation of the characters in the corrected image, and use the threading method to perform character recognition on the independently segmented image, and then use the regex filtering module to extract the key information from the recognition text, and calculates the average and difference values of the blood pressure measurement readings.

6. The method of claim 1, wherein the specific process of using the text detection module and the text recognition module to detect and recognize the textual information in the processed document image in the step S23 is as follows:
text detection: using the DBnet network to realize text detection, outputting the text segmentation result of the image through the network based on the segmentation method, using the preset threshold T to convert the segmentation result image into a binary image, inserting the image binarization operation into the network for joint optimization, through more than 4 sampling operations, the network adaptively predicts the threshold of each pixel point in the image, using the ICPR dataset to train the document information model, parsing the text into a list and storing it, then performing recognition after training;
text recognition: the recognition algorithm is implemented by CRNN+CTC, by extracting features, the text sequence features are extracted based on convolution features, and selecting GRU with lower computational cost; CNN uses Mobilenet to scale to the size of 32x640x32 under the case of fixed aspect ratio of the image, which becomes 1x8x512 after passing through the CNN; setting T=8 for LSTM, and inputting the features into LSTM, in the actual recognition process, taking into account the text repetition, interruption, and different intervals of document information, using CTC to output the results, borrowing the forward-backward algorithm of HMM to predict the blanks in the text by human-specified blank symbols, finally enumerating all possible results, and outputting the recognition text information.

7. The method of claim 2, wherein the specific process of using the preset threshold T to convert the segmentation result image into a binary image and the threshold T is obtained by using the OTSU threshold segmentation algorithm is as follows:
① the size of the blood pressure measurement result image P is width w, height h, the segmentation threshold is T, and T is traversed from 0 to 255;
② dividing the image into two parts according to the size of T, and the number of pixels smaller than the threshold T is *N*₀ and the number of pixels greater than the threshold T is *N*₁, wherein *N*₀ + *N₁* = *w* * *h;*
(3) the proportions of the two divided parts to the entire image P are *ω*₀ and *ω*₁, *ω*₀ + *ω*₁ = 1; the average gray level are µ₀ and µ₁ separately, wherein *µ*₀ + *µ*₁ = 1;
④ the average gray value of the image P is *µ*, with a variance of *ε*, *ε* = *ω*₀(*µ*₀ - *µ*)² + *ω*₁(*µ*₁ - *µ*)², according to the OTSU algorithm, it can be derived:
*ε* = *ω*₀*ω*₁(*µ*₀ - *µ*₁)², wherein *ω*₀ = *N₀*/*(w* * *h), ω₁* = *N₁*/*(w * h*), *µ* = *µ*₀*ω*₀ + *µ*₁*ω*₁*;*
by traversing the pixels of the image P, determining the size of the threshold T, that is, the size of the threshold T based on the maximum value of the variance ε.
